# EUROPEAN PATENT SPECIFICATION

(11) **EP 2 744 347 B1**
(45) Date of publication and mention of the grant of the patent: **07.08.2019**
(21) Application number: 12823900.1
(22) Date of filing: 15.08.2012
(51) Int. Cl.: A01P 13/00, A01N 39/02, A01N 39/04, A01N 43/40, A01N 25/02, A01N 25/04, A01N 25/24, A01N 25/32

(54) **COMPLEXES OF HERBICIDAL CARBOXYLIC ACIDS AND AMINE-CONTAINING POLYMERS OR OLIGOMERS**
KOMPLEXE AUS HERBIZIDEN CARBONSÄUREN UND AMINENTHALTENDEN POLYMEREN ODER OLIGOMEREN
COMPLEXES D'ACIDES CARBOXYLIQUES HERBICIDES ET DE POLYMÈRES OU OLIGOMÈRES À TENEUR EN AMINE

(30) Priority: 16.08.2011 US 201161523958 P
(43) Date of publication of application: 25.06.2014
(73) Proprietor: Dow AgroSciences LLC, Indianapolis, IN 46268 (US)
(72) Inventor: LIU, Lei, Carmel, IN 46032 (US); ZHANG, Hong, Carmel, IN 46032 (US); KENNEDY, Alex, Indianapolis, IN 46260 (US); TANK, Holger, Zionsville, IN 46077 (US); OUSE, David, G., Indianapolis, IN 46256 (US); GIFFORD, James, M., Lebanon, IN 46052 (US); ZHAO, Min, Westfield, IN 46074 (US)
(74) Representative: f & e patent
(86) International application number: PCT/US2012/050871
(87) International publication number: WO 2013/025758

(56) References cited:
- EP-A1- 0 375 624
- WO-A1-99/05914
- WO-A1-2005/087007
- WO-A1-2008/069826
- WO-A1-2011/040956
- WO-A1-2012/027349
- WO-A1-2012/059494
- WO-A1-2013/189773
- WO-A2-91/04661
- WO-A2-2009/076349
- WO-A2-2009/141367
- WO-A2-2011/019652
- WO-A2-2011/039172
- WO-A2-2012/076567
- US-A- 5 527 524
- US-A- 6 121 200
- US-A1- 2002 107 149
- US-A1- 2002 160 916
- US-A1- 2006 040 828
- US-A1- 2007 149 409
- US-A1- 2008 085 983
- US-A1- 2008 182 773
- DATABASE CA [Online] CHEMICAL ABSTRACTS SERVICE, COLUMBUS, OHIO, US; AKAGANE, KATSUO ET AL: "Long-term protection. II. Effects of fungicidal polymers for long-term protection", XP002736669, retrieved from STN Database accession no. 77:165971 & AKAGANE, KATSUO ET AL: "Long-term protection. II. Effects of fungicidal polymers for long-term protection", SHIKIZAI KYOKAISHI , 45(9), 479-84 CODEN: SKYOAO; ISSN: 0010-180X, 1972,

## Description

### BACKGROUND

Compositions containing herbicidal and plant growth modifying chemicals are widely used in agricultural, industrial, recreational, and residential areas worldwide. The active ingredients of such compositions are frequently carboxylic acids, more particularly their salts. These carboxylic acid salts generally have very high water solubility leading to their use in high strength aqueous concentrates intended for dilution in water prior to application by spraying or other means.

Carboxylic acid herbicides such as the synthetic auxin herbicide 2,4-dichlorophenoxyacetic acid (2,4-D) have long been used to control unwanted vegetation. 2,4-D is normally converted into liquid formulations by conversion to water soluble salts or emulsifiable esters. The ester formulations have been found to be more effective than the salts on an acid equivalent basis in the control of noxious vegetation, but have the unwanted characteristic of migrating to adjacent desirable vegetation because of their volatility, resulting in unacceptable damage to nearby sensitive plants.

Efforts to solve the volatility problem, including preparation of water soluble salts such as the dimethylamine salt of 2,4-D, have not been totally satisfactory because, upon volatilization of the amine, the herbicide reverts back to its initial acid form, which, in itself under certain unfavorable conditions, has sufficient volatility to migrate and cause damage to nearby sensitive crops.

Formulations comprising a pesticide or a salt thereof and a polymeric polyamine are described e.g. in US 2007/0149409 A1, WO 2011/019652 A2, WO 2012/027349 A1, WO 2011/040956 A1 and by K. Akagane et al. Shikizai Kyokaishi 1972, 45(9), 479-484.

While there are many herbicide products available for weed control in both crop and non-crop markets, there is a need for new herbicide products that offer improved performance and cost efficiencies to the end user. Described herein are novel compositions containing herbicidal carboxylic acids that provide improved weed efficacy and low volatility.

### SUMMARY

Herbicidal compositions including 10 to 50 wt.-% on an acid equivalent basis of a complex of a herbicidal carboxylic acid and an amine-containing polymer or oligomer, an organic solvent and optionally one or more inert ingredients are described. Herein, the amine-containing polymer or oligomer is a polyethyleneimine with a molecular weight of greater than 250 and less than 2,000,000 and a nitrogen content from 10 to 50 wt.-%. The herbicidal compositions have herbicidal activity on an acid equivalent (AE) basis that is equivalent to or better than the commercially used carboxylic acid herbicide salts. The herbicidal activity of the herbicidal compositions described herein is also, on an AE basis, comparable to the highly active and, in some cases, volatile ester derivatives of these herbicidal carboxylic acids. The herbicidal compositions described herein additionally have lower volatility as compared to known salt compositions and exhibit lower soil mobility which provide for the protection of nearby sensitive crops. The herbicidal compositions described herein are emulsifiable concentrates which readily form stabilized emulsions when mixed with water that are useful in broadcast spray applications, or the diluted compositions obtained by diluting said emulsifiable concentrates with an inert carrier.

A method of controlling undesirable vegetation including the step of contacting the vegetation or the locus thereof with, or applying to the soil to prevent the emergence of vegetation, a herbicidally effective amount of the herbicidal composition described herein also is described.

Additionally, a method of reducing the volatility of a herbicidal carboxylic acid by adding an amine-containing polymer or oligomer to the herbicidal carboxylic acid also is described.

Further, a method of reducing the soil mobility of a herbicidal carboxylic acid by adding an amine-containing polymer or oligomer to the herbicidal carboxylic acid also is described.

### BRIEF DESCRIPTION OF THE FIGURES

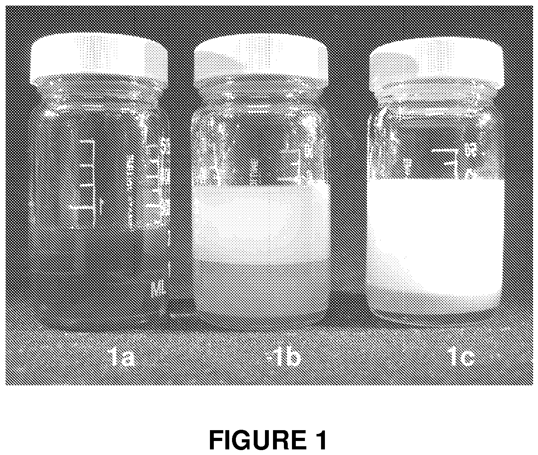

Figure 1 shows images of a complex of 2,4-D and Lupasol® G20 polyethyleneimine as a 50 wt% acid equivalent (AE) concentrate in Dowanol® EB (left, Example Solution 1a in Table 11), as a 20 wt% AE mixture of the concentrate diluted in water (center, Example Solution 1b in Table 11), and as a 10 wt% AE mixture of the concentrate diluted in water (right, Example Solution 1c in Table 11).

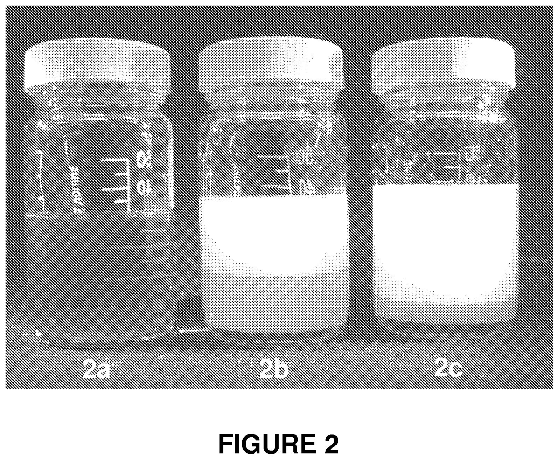

Figure 2 shows images of a complex of 2,4-D and Lupasol® FG polyethyleneimine as a 50 wt% acid equivalent (AE) concentrate in Dowanol® EB (left, Example Solution 2a in Table 11), as a 20 wt% AE mixture of the concentrate diluted in water (center, Example Solution 2b in Table 11), and as a 10 wt% AE mixture of the concentrate diluted in water (right, Example Solution 2c in Table 11).

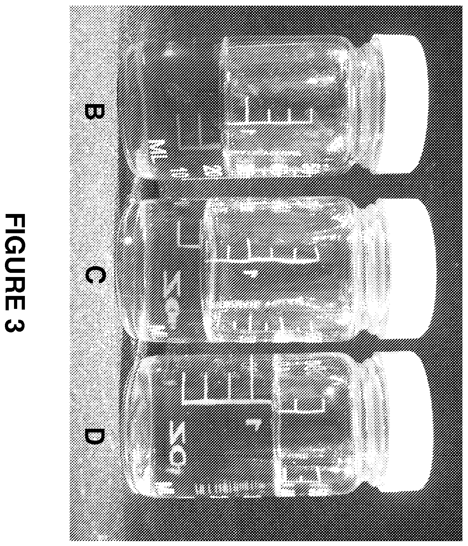

Figure 3 shows images of a 2,4-D dimethylamine (DMA) salt with added Lupasol®G20 polyethyleneimine (10 wt%) as a 40 wt% AE aqueous concentrate (left, Comparative Solution B in Table 11), as a 20 wt% AE solution in water prepared by diluting the concentrate (center, Comparative Solution C in Table 11), and as a 10 wt% AE solution in water prepared by diluting the concentrate (right, Comparative Solution D in Table 11).

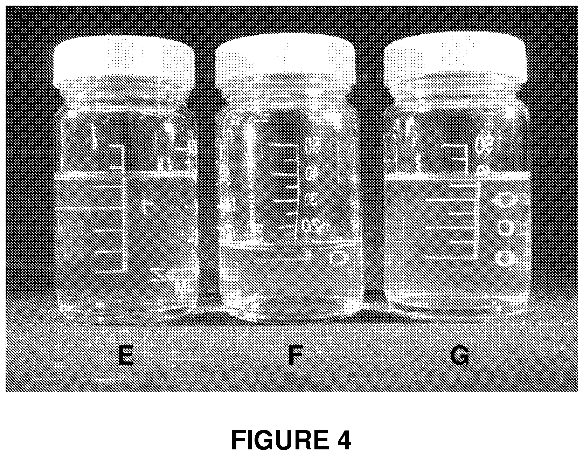

Figure 4 shows images of a 2,4-D TEPA salt as a 21.1% AE solution in water containing 6.5 wt% TEPA (left, Comparative Solution E in Table 11), a 20% AE solution in water prepared by diluting the 21.1 wt% AE solution (center, Comparative Solution F in Table 11), and a 10% AE solution in water prepared by diluting the 21.1 wt% AE solution (right, Comparative Solution G in Table 11).

### DETAILED DESCRIPTION

Complexes of herbicidal carboxylic acids and amine-containing polymers or oligomers are described herein. These herbicidal complexes are provided in form of an emulsifiable concentrate according to claim 1 or a diluted composition obtained by diluting said emulsifiable concentrate with an inert carrier according to claim 6. These herbicidal complexes can further be provided in admixture with agriculturally acceptable adjuvants and/or carriers.

The herbicidal carboxylic acids described herein include aryloxyalkanoic acid compounds of the following general formula 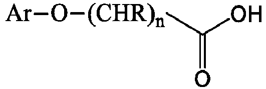 wherein R is independently H or CH₃, n is an integer 1, 2 or 3, and Ar is a phenyl or pyridine group substituted with one or more substituents selected from halogen, C₁-C₆ alkyl, C₁-C₆ haloalkyl, C₁-C₆ alkoxy, amino, C₁-C₆ alkylamino, and di(C₁-C₆ alkyl)amino.

The herbicidal carboxylic acids described herein also include aryloxyphenoxypropionic acid compounds of the following general formula 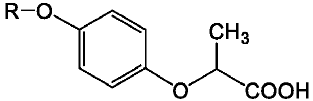 and stereoisomers thereof, wherein R is a substituted phenyl, pyridine, benzoxazole, or quinoxaline group with one or more substituents selected from halogen, C₁-C₆ haloalkyl, or cyano.

Suitable herbicidal carboxylic acids include *pyridinyloxyacetic acid herbicides* such as fluroxypyr and triclopyr; *phenoxyacetic herbicides* such as 4-CPA, 2,4-D, 3,4-DA, and MCPA; *phenoxybutyric acid herbicides* such as 4-CPB, 2,4-DB, 3,4-DB, and MCPB; *phenoxypropionic acid herbicides* such as cloprop, 4-CPP, dichlorprop, 3,4-DP, fenoprop, mecoprop, and mecoprop-P; and *aryloxyphenoxypropionic acid herbicides* such as chlorazifop, clodinafop, clofop, cyhalofop, diclofop, fenoxaprop, fluazifop, haloxyfop, quizalofop, and trifop. Preferred herbicidal carboxylic acids include 2,4-D, 2,4-DB, triclopyr, fluroxypyr, MCPA, MCPB, mecoprop, mecoprop-P, cyhalofop, fluazifop, haloxyfop, clodinafop, quizalofop, and fenoxaprop.

As used herein the term complex refers to a substance formed by the union of simpler substances that are held together by non-covalent bonding forces such as, but not limited to, hydrogen bonding, ionic bonding, or dipole-dipole interactions.

As used herein the chemical term salt refers to a substance composed of one or more cations (i.e., positively charged ions) and one or more anions (i.e., negatively charged ions) such that the substance is electrically neutral (i.e., without a net charge). The component ions of a salt can be inorganic such as chloride (Cl⁻) or organic such as acetate (CH₃COO⁻). Salts can be formed by a neutralization reaction between an acid and a base and generally have good solubility in water. Aqueous solutions containing salts normally exhibit high electrical conductivity due to the dissociative nature of the component ions in solution.

The electrical conductivity of a solution is a measure of the flow of positively and negatively charged ions (i.e., cations and anions) when an electric potential is applied. As a simple example, if an electric potential is applied across an aqueous solution containing sodium (Na⁺) and chloride (Cl⁻) ions, the sodium ions will move towards the negative electrode (cathode) and the chloride ions will move towards the positive electrode (anode) completing a circuit through the solution. Electrical conductivity is generally measured in units of siemens (S) per unit length such as siemens per meter (S/m; SI units are kg⁻¹·m³·s³·A²) or millisiemens/centimeter (mS/cm). Generally, solutions with a high electrical conductivity value contain compounds with a high ionic/dissociative character and solutions with a low electrical conductivity value contain compounds that are not dissociating into ionic species.

The complexes of herbicidal carboxylic acids and amine-containing polymers or oligomers described herein are described as complexes rather than salts because of their distinctive physical properties. The complexes described herein exhibit less than 10 weight percent (wt%) solubility in water, preferably less than 5 wt% solubility in water, and most preferably less than one wt% solubility in water (solubility being measured at room temperature and at a pH of 7). The complexes described herein have good to excellent solubility in polar organic solvents and form emulsions when added to water as a solution in a polar organic solvent. The lower water solubility of the complexes described herein combined with their observed lower conductivity (compared to the respective salts) in water indicate that the complexes described herein do not behave as salts, but instead have a distinct character described herein as being that of a complex where the component parts of the complex, the herbicidal carboxylic acid, and the amine-containing polymer or oligomer, do not significantly dissociate from one another and form ions in solution, but instead stay in close association. In addition, the compositions described herein, in many instances, exhibit herbicidal efficacy that is similar to known herbicidal carboxylic acid ester derivatives and more efficacious than known herbicidal carboxylic acid amine salts.

Amine-containing polymers and oligomers used with the complexes described herein in the claimed formulations include those polymers and oligomers that have a molecular weight (mw) from 250 to 2,000,000, *e.g.* from 300 to 2,000,000, from 350 to 2,000,000, from 400 to 2,000,000, from 450 to 2,000,000, from 500 to 2,000,000, from 550 to 2,000,000, from 600 to 2,000,000, from 650 to 2,000,000, from 700 to 2,000,000, from 750 to 2,000,000, from 800 to 2,000,000, from 850 to 2,000,000, from 900 to 2,000,000, from 950 to 2,000,000, from 1000 to 2,000,000, from 1050 to 2,000,000, from 1100 to 2,000,000, from 1150 to 2,000,000, and from 1200 to 2,000,000 Daltons. Preferably the amine-containing polymers and oligomers useful with the complexes described herein include polymers and oligomers that have a molecular weight (mw) from 500 to 1,000,000, from 550 to 1,000,000, from 600 to 1,000,000, from 650 to 1,000,000, from 700 to 1,000,000, from 750 to 1,000,000, from 800 to 1,000,000, from 850 to 1,000,000, from 900 to 1,000,000, from 950 to 1,000,000, from 1000 to 1,000,000, from 1050 to 1,000,000, from 1100 to 1,000,000, from 1150 to 1,000,000, and from 1200 to 1,000,000 Daltons. Most preferably the amine-containing polymers and oligomers useful with the complexes described herein include polymers and oligomers that have a molecular weight (mw) from 500 to 10,000, from 550 to 10,000, from 600 to 10,000, from 650 to 10,000, from 700 to 10,000, from 750 to 10,000, from 800 to 10,000, from 850 to 10,000, from 900 to 10,000, from 950 to 10,000, from 1000 to 10,000, from 1050 to 10,000, from 1100 to 10,000, from 1150 to 10,000, and from 1200 to 10,000 Daltons.

The amine-containing polymers or oligomers used with the complexes described herein in the claimed formulations have a nitrogen content of from 10 to 50 percent by weight.

The polyethyleneimines used with the complexes described herein in the claimed formulations may include linear or branched-chain polyethyleneimine polymers or oligomers including 10 or more monomer units, and derivatives, analogs, co-polymers, and mixtures thereof. Preferred polyethyleneimines include branched, spherical polyamines with a well-defined ratio of primary, secondary, and tertiary amine functional groups. These polyethyleneimines can be best described by the following partial structural formula: 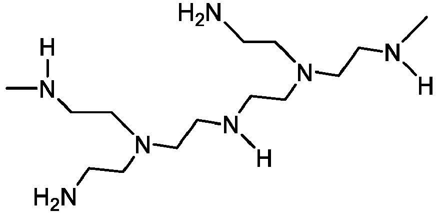 The polyethyleneimines used with the complexes described herein in the claimed formulations can be prepared by the polymerization of ethyleneimine. Examples of commercially available polyethyleneimines include the Lupasol® or Epomin® families of products such as, for example, Lupasol® G20, Lupasol® FG, Lupasol® G35, Lupasol® P, and Lupasol® 1595 (the Lupasol® series of products are available from BASF (Florham Park, NJ)), and Epomin® SP-003, Epomin® SP-006, Epomin® SP-012, Epomin® SP-018, Epomin® SP-200, Epomin® SP-1000, and Epomin® SP-1050 (the Epomin® series of products are available from Nippon Shokubai (Osaka, Japan)).

The relative amounts of the amine-containing polymer or oligomer and the herbicidal carboxylic acid used in the complexes of herbicidal carboxylic acids described herein can be described by the molar ratio of the amine groups to the carboxylic acid groups contained in the polymeric complexes of the herbicidal carboxylic acids. For example, the molar ratio of amine groups to carboxylic acid groups useful in the complexes of herbicidal carboxylic acids described herein may range from 5:1 to 1:5, preferably from 2:1 to 1:2. The molar ratio may be 5:1, 4:1, 3:1, 2:1, 1:1, 1:2, 1:3, 1:4, or 1:5 and incremental values between these ratios. For example, if one mole of amine-containing polymer or oligomer molecules containing an average of 30 amine groups per polymer or oligomer molecule were combined with 30 moles of 2,4-D acid, a polymeric complex of the amine-containing polymer or oligomer and 2,4-D acid would be formed where the molar ratio of amine groups to carboxylic acid groups would be 1:1.

The polymeric complexes of herbicidal carboxylic acids as described herein are formed by combining a herbicidal carboxylic acid with an amine-containing polymer or oligomer. The compositions containing the polymeric complexes are formulated as emulsifiable concentrates (EC). These properties allow the compositions of polymeric complexes of herbicidal carboxylic acids described herein to be emulsified in a carrier such as water for use in spray applications such as, for example, to control unwanted plant growth. Suitable organic solvents useful for preparing and storing the compositions of polymeric complexes of herbicidal carboxylic acids described herein generally are polar in nature and may include, but are not limited to, alcohols such as methanol, ethanol, ethylene glycol, and propylene glycol, derivatives of ethylene and propylene glycol such as alkylated ethylene glycols and oligomers thereof such as Dowanol® EB, DB, TBH, DM, and TMH (the Dowanol ® series of products are available from The Dow Chemical Company (Midland, MI)), and propylene glycol butyl ether; ketones such as acetone, acetophenone, cyclohexanone, methyl ethyl ketone, and methyl iso-butyl ketone; sulfoxides or sulfones such as dimethyl sulfoxide and sulfolane; ethers such as tetrahydrofuran and dioxane; nitriles such as acetonitrile and butyronitrile; *N,N*-dialkyl amides such as, *N*-methyl-2-pyrrolidinone and *N,N*-dimethyl alkylamides; esters such as butyl lactate, and mixtures thereof, and mixtures of any of the above solvents with water.

The compositions of polymeric complexes of herbicidal carboxylic acids described herein can be prepared by combining the herbicidal carboxylic acid with the amine-containing polymer, with the aid of a polar organic solvent and optionally additional inert formulation ingredients.

Also described herein is a method of controlling undesirable vegetation by contacting the vegetation or the locus thereof with or applying to the soil to prevent emergence of the vegetation a herbicidally effective amount of a diluted composition obtained by diluting the emulsifiable concentrate described herein with an inert carrier. The compositions described herein offer improved herbicidal efficacy, on an acid equivalent (AE) basis, when compared to existing alkylamine salt compositions of the herbicidal carboxylic acids such as, for example, 2,4-D dimethylamine, and the compositions described herein are comparable in activity to the highly herbicidally active ester derivatives of the herbicidal carboxylic acids.

Further described herein is a method of reducing the volatility of herbicidal carboxylic acids in spray applications for the control of unwanted plant growth by the use of the compositions described herein. Preferred herbicidal carboxylic acids that exhibit reduced volatility by their conversion into the compositions described herein are the synthetic auxin herbicides such as *phenoxyacetic acid herbicides, phenoxybutyric acid herbicides, phenoxypropionic acid herbicide,* and *pyridinyloxyacetic acid herbicides.* Especially preferred herbicidal carboxylic acids that exhibit reduced volatility by their conversion into the compositions described herein are 2,4-D, 2,4-DB, MCPA, MCPB, mecoprop, mecoprop-P, and triclopyr.

Described herein is a hi-load emulsifiable concentrate that includes, with respect to the total composition, from 10 to 50 weight percent (wt%) on an acid equivalent (AE) basis of the compositions described herein, an organic solvent, and, optionally, one or more additional inert ingredients. Preferred herbicidal carboxylic acids for use in such a concentrate include 2,4-D, 2,4-DB, MCPA, MCPB, mecoprop, mecoprop-P, triclopyr, and fluroxypyr. Such a concentrate upon dilution in water forms a stable, homogeneous emulsion that is readily used in spray applications to control plant growth.

The compositions described herein also bind to soil more readily than their corresponding salt derivatives which reduces the mobility in soil of the herbicidal carboxylic acid and therefore reduces the potential for movement of the herbicidal carboxylic acid into surface and ground water, and thereby reduces the potential of the herbicidal carboxylic acid to cause damage to adjacent non-target plants.

The compositions described herein also offer improved binding to plant surfaces such as leaves and stems, and thereby provide improved deposition and residuality of the compositions on those surfaces. The improved plant surface binding also improves the residuality or rain fastness of the compositions described herein in high humidity or during high surface moisture conditions such as during rains or dews.

The compositions described herein also offer a decreased potential for eye irritation compared to the commonly used alkyl ammonium salt derivatives of the herbicidal carboxylic acids. One disadvantage of using alkyl ammonium salts of herbicidal carboxylic acids to prepare aqueous herbicide concentrates is that the concentrates and solutions made using the concentrates can be irritating to the eyes of anyone handling the concentrates upon exposure, e.g., by splashing into the eye. This disadvantage may lead to restrictive labeling of the products that limits their usefulness in certain markets, even if the active ingredient itself provides no such hazard. Because the compositions described herein are complexes and do not have the physical properties of salts such as, for example, high water solubility and high conductivity in water, they offer a lower potential to cause eye irritation.

The compositions described herein also offer control of or reduction of off-target spray drift during agricultural spray applications to control unwanted plants. By incorporating the compositions described herein into a spray solution or mixture, the amount of driftable fines of the spray in both aerial and ground spray applications is reduced. The negative consequences of off-target spray movement can be quite pronounced. Some herbicides have demonstrated very sensitive phytotoxicity to particular plant species at extremely low parts per million (ppm) or even parts per billion (ppb) levels, resulting in restricted applications around sensitive crops, orchards, and residential plantings.

The compositions described herein can also be used in combination with other agricultural active ingredients such as, for example, herbicides, insecticides, fungicides, plant growth regulators, herbicide safeners, various mixtures and combinations of these, and the like. These combinations may be pre-mix concentrates or spray solutions prepared by either diluting such a concentrate or tank-mixing the components of the spray solution, or they may be applied sequentially with the other agricultural active ingredient or ingredients.

Herbicides that may be employed in conjunction with the compositions described herein include, but are not limited to, 2,4-DEB, 2,4-DEP, 2,3,6-TBA, acetochlor, acifluorfen, aclonifen, acrolein, alachlor, allidochlor, alloxydim, allyl alcohol, alorac, ametridione, ametryn, amibuzin, amicarbazone, amidosulfuron, aminocyclopyrachlor, aminopyralid, amiprofos-methyl, amitrole, ammonium sulfamate, anilofos, anisuron, asulam, atraton, atrazine, azafenidin, azimsulfuron, aziprotryne, barban, BCPC, beflubutamid, benazolin, bencarbazone, benfluralin, benfuresate, bensulfuron, bensulide, bentazone, benzadox, benzfendizone, benzipram, benzobicyclon, benzofenap, benzofluor, benzoylprop, benzthiazuron, bicyclopyrone, bifenox, bilanafos, bispyribac, borax, bromacil, bromobonil, bromobutide, bromofenoxim, bromoxynil, brompyrazon, butachlor, butafenacil, butamifos, butenachlor, buthidazole, buthiuron, butralin, butroxydim, buturon, butylate, cacodylic acid, cafenstrole, calcium chlorate, calcium cyanamide, cambendichlor, carbasulam, carbetamide, carboxazole chlorprocarb, carfentrazone, CDEA, CEPC, chlomethoxyfen, chloramben, chloranocryl, chlorazine, chlorbromuron, chlorbufam, chloreturon, chlorfenac, chlorfenprop, chlorflurazole, chlorflurenol, chloridazon, chlorimuron, chlornitrofen, chloropon, chlorotoluron, chloroxuron, chloroxynil, chlorpropham, chlorsulfuron, chlorthal, chlorthiamid, cinidon-ethyl, cinmethylin, cinosulfuron, cisanilide, clethodim, cliodinate, clomazone, cloproxydim, clopyralid, cloransulam, CMA, copper sulfate, CPMF, CPPC, credazine, cresol, cumyluron, cyanatryn, cyanazine, cycloate, cyclosulfamuron, cycloxydim, cycluron, cyperquat, cyprazine, cyprazole, cypromid, daimuron, dalapon, dazomet, delachlor, desmedipham, desmetryn, di-allate, dicamba, dichlobenil, dichloralurea, dichlormate, diclosulam, diethamquat, diethatyl, difenopenten, difenoxuron, difenzoquat, diflufenican, diflufenzopyr, dimefuron, dimepiperate, dimethachlor, dimethametryn, dimethenamid, dimethenamid-P, dimexano, dimidazon, dinitramine, dinofenate, dinoprop, dinosam, dinoseb, dinoterb, diphenamid, dipropetryn, diquat, disul, dithiopyr, diuron, DMPA, DNOC, DSMA, EBEP, eglinazine, endothal, epronaz, EPTC, erbon, esprocarb, ethalfluralin, ethametsulfuron, ethidimuron, ethiolate, ethofumesate, ethoxyfen, ethoxysulfuron, etinofen, etnipromid, etobenzanid, EXD, fenasulam, fenoxasulfone, fenteracol, fentrazamide, fenuron, ferrous sulfate, flamprop, flamprop-M, flazasulfuron, florasulam, fluazolate, flucarbazone, flucetosulfuron, fluchloralin, flufenacet, flufenican, flufenpyr, flumetsulam, flumezin, flumiclorac, flumioxazin, flumipropyn, fluometuron, fluorodifen, fluoroglycofen, fluoromidine, fluoronitrofen, fluothiuron, flupoxam, flupropacil, flupropanate, flupyrsulfuron, fluridone, flurochloridone, flurtamone, fluthiacet, fomesafen, foramsulfuron, fosamine, furyloxyfen, glufosinate, glufosinate-P, glyphosate, halosafen, halosulfuron, haloxydine, hexachloroacetone, hexaflurate, hexazinone, imazamethabenz, imazamox, imazapic, imazapyr, imazaquin, imazethapyr, imazosulfuron, indanofan, indaziflam, iodobonil, iodomethane, iodosulfuron, iofensulfuron, ioxynil, ipazine, ipfencarbazone, iprymidam, isocarbamid, isocil, isomethiozin, isonoruron, isopolinate, isopropalin, isoproturon, isouron, isoxaben, isoxachlortole, isoxaflutole, karbutilate, ketospiradox, lactofen, lenacil, linuron, MAA, MAMA, medinoterb, mefenacet, mefluidide, mesoprazine, mesosulfuron, mesotrione, metam, metamitron, metazachlor, metazosulfuron, metflurazon, methabenzthiazuron, methalpropalin, methazole, methiobencarb, methiozolin, methiuron, methometon, methoprotryne, methyl bromide, methyl isothiocyanate, methyldymron, metobenzuron, metobromuron, metolachlor, metosulam, metoxuron, metribuzin, metsulfuron, molinate, monalide, monisouron, monochloroacetic acid, monolinuron, monuron, morfamquat, MSMA, naproanilide, napropamide, naptalam, neburon, nicosulfuron, nipyraclofen, nitralin, nitrofen, nitrofluorfen, norflurazon, noruron, OCH, orbencarb, *ortho-*dichlorobenzene, orthosulfamuron, oryzalin, oxadiargyl, oxadiazon, oxapyrazon, oxasulfuron, oxaziclomefone, oxyfluorfen, parafluron, paraquat, pebulate, pelargonic acid, pendimethalin, penoxsulam, pentachlorophenol, pentanochlor, pentoxazone, perfluidone, pethoxamid, phenisopham, phenmedipham, phenmedipham-ethyl, phenobenzuron, phenylmercury acetate, picloram, picolinafen, pinoxaden, piperophos, potassium arsenite, potassium azide, potassium cyanate, pretilachlor, primisulfuron, procyazine, prodiamine, profluazol, profluralin, profoxydim, proglinazine, prometon, prometryn, propachlor, propanil, propazine, propham, propisochlor, propoxycarbazone, propyrisulfuron, propyzamide, prosulfalin, prosulfocarb, prosulfuron, proxan, prynachlor, pydanon, pyraclonil, pyraflufen, pyrasulfotole, pyrazolynate, pyrazosulfuron, pyrazoxyfen, pyribenzoxim, pyributicarb, pyriclor, pyridafol, pyridate, pyriftalid, pyriminobac, pyrimisulfan, pyrithiobac, pyroxasulfone, pyroxsulam, quinclorac, quinmerac, quinoclamine, quinonamid, rhodethanil, rimsulfuron, saflufenacil, S-metolachlor, sebuthylazine, secbumeton, sethoxydim, siduron, simazine, simeton, simetryn, SMA, sodium arsenite, sodium azide, sodium chlorate, sulcotrione, sulfallate, sulfentrazone, sulfometuron, sulfosulfuron, sulfuric acid, sulglycapin, swep, TCA, tebutam, tebuthiuron, tefuryltrione, tembotrione, tepraloxydim, terbacil, terbucarb, terbuchlor, terbumeton, terbuthylazine, terbutryn, tetrafluron, thenylchlor, thiazafluron, thiazopyr, thidiazimin, thidiazuron, thiencarbazone-methyl, thifensulfuron, thiobencarb, tiocarbazil, tioclorim, topramezone, tralkoxydim, triafamone, tri-allate, triasulfuron, triaziflam, tribenuron, tricamba, tridiphane, trietazine, trifloxysulfuron, trifluralin, triflusulfuron, trifopsime, trihydroxytriazine, trimeturon, tripropindan, tritac, tritosulfuron, vernolate, and xylachlor.

Especially suitable combinations may comprise the compositions described herein used in combination with one or more of aminopyralid, dicamba, florasulam, glyphosate, glufosinate, and picloram.

The compositions described herein can additionally be employed to control undesirable vegetation in many crops that have been made tolerant to or resistant to them or to other herbicides by genetic manipulation or by mutation and selection. The compositions described herein can, further, be used in conjunction with glyphosate, glufosinate, dicamba, or imidazolinones on glyphosate-tolerant, glufosinate-tolerant, dicamba-tolerant, imidazolinone-tolerant, or 2,4-D-tolerant crops. The compositions described herein are preferably used in combination with herbicides that are selective for the crop being treated and complement the spectrum of weeds controlled by these compounds at the application rate employed. The compositions described herein are preferably applied at the same time as other complementary herbicides, either as a combination formulation or as a tank mix. Similarly the compositions described herein can be used in conjunction with acetolactate synthase inhibitors on acetolactate synthase inhibitor tolerant crops.

The compositions described herein can generally be employed in combination with known herbicide safeners, such as benoxacor, benthiocarb, brassinolide, cloquintocet (mexyl), cyometrinil, daimuron, dichlormid, dicyclonon, dimepiperate, disulfoton, fenchlorazole-ethyl, fenclorim, flurazole, fluxofenim, furilazole, harpin proteins, isoxadifen-ethyl, mefenpyr-diethyl, MG 191, MON 4660, naphthalic anhydride (NA), oxabetrinil, R29148, and *N*-phenylsulfonylbenzoic acid amides, to enhance their selectivity. They can additionally be employed to control undesirable vegetation in many crops that have been made tolerant to or resistant to them or to other herbicides by genetic manipulation or by mutation and selection. For example, corn, wheat, rice, soybean, sugarbeet, cotton, canola, and other crops that have been made tolerant or resistant to the compositions described herein in sensitive plants can be treated. Some crops (e.g. cotton) have been made tolerant to auxinic herbicides such as 2,4-dichlorophenoxyacetic acid. The compositions described herein derived from auxin herbicides may be used to treat such resistant crops or other auxin herbicide tolerant crops.

The term herbicide is used herein to mean an active ingredient that kills, controls, or otherwise adversely modifies the growth of plants. A herbicidally effective or vegetation controlling amount is an amount of active ingredient which causes an adversely modifying effect and includes deviations from natural development, killing, regulation, desiccation, retardation, and the like. The terms plants and vegetation include germinant seeds, emerging seedlings, and established vegetation.

Herbicidal activity is exhibited by the compositions described herein when is the compositions are applied directly to the plant or to the locus of the plant at any stage of growth or before planting or emergence. The effect observed depends upon the plant species to be controlled, the stage of growth of the plant, the application parameters of dilution and spray drop size, the particle size of solid components, the environmental conditions at the time of use, the specific compound employed, the specific adjuvants and carriers employed, the soil type, and the like, as well as the amount of chemical applied. These and other factors can be adjusted as is known in the art to promote non-selective or selective herbicidal action.

Application rates of 1 to 2,000 grams per hectare (g/ha) are generally employed in both postemergence and preemergence applications. The higher rates designated generally give non-selective control of a broad variety of undesirable vegetation. The lower rates typically give selective control and can be employed in the locus of crops.

While it is possible to utilize the compositions described herein directly as herbicides, it is preferable to use them in mixtures containing a herbicidally effective amount of the compositions described herein along with at least one agriculturally acceptable adjuvant or carrier. Suitable adjuvants or carriers should not be phytotoxic to valuable crops, particularly at the concentrations employed in applying the compositions for selective weed control in the presence of crops, and should not react chemically with the compositions described herein or other composition ingredients. Such mixtures can be designed for application directly to weeds or their locus or can be concentrates or formulations that are normally diluted with additional carriers and adjuvants before application.

Suitable agricultural adjuvants and carriers that are useful in preparing the compositions of polymeric complexes of herbicidal carboxylic acids described herein are well known to those skilled in the art.

Liquid carriers that can be employed include water and organic solvents. The organic solvents typically used include, but are not limited to, petroleum fractions or hydrocarbons such as mineral oil, aromatic solvents, paraffinic oils, and the like; vegetable oils such as soy bean oil, rape seed oil, olive oil, castor oil, sunflower seed oil, coconut oil, corn oil, cotton seed oil, linseed oil, palm oil, peanut oil, safflower oil, sesame oil, tung oil, and the like; esters of the above vegetable oils; esters of monoalcohols or dihydric, trihydric, or other lower polyalcohols (4-6 hydroxy containing), such as 2-ethyl hexyl stearate, n-butyl oleate, isopropyl myristate, propylene glycol dioleate, di-octyl succinate, di-butyl adipate, di-octyl phthalate and the like; esters of mono, di and polycarboxylic acids, and the like. Specific organic solvents include toluene, xylene, petroleum naphtha, crop oil, acetone, methyl ethyl ketone, cyclohexanone, trichloroethylene, perchloroethylene, ethyl acetate, amyl acetate, butyl acetate, propylene glycol monomethyl ether and diethylene glycol monomethyl ether, methanol, ethanol, isopropanol, amyl alcohol, ethylene glycol, propylene glycol, glycerine, and the like. Water is generally the carrier of choice for the dilution of concentrates.

Suitable solid carriers include talc, pyrophyllite clay, silica, attapulgus clay, kaolin clay, kieselguhr, chalk, diatomaceous earth, lime, calcium carbonate, bentonite clay, Fuller's earth, cottonseed hulls, wheat flour, soybean flour, pumice, wood flour, walnut shell flour, lignin, and the like.

Surface-active agents can be incorporated into the compositions described herein. Such surface-active agents are advantageously employed in both solid and liquid compositions, especially those designed to be diluted with carrier before application. The surface-active agents can be anionic, cationic, or nonionic in character and can be employed as emulsifying agents, wetting agents, suspending agents, or for other purposes. Surfactants conventionally used in the art of formulation and which may also be used in the compositions described herein are described, inter alia, in "McCutcheon's Detergents and Emulsifiers Annual", MC Publishing Corp., Ridgewood, New Jersey, 1998 and in "Encyclopedia of Surfactants", Vol. I-III, Chemical publishing Co., New York, 1980-81. Typical surface-active agents include salts of alkyl sulfates, such as diethanolammonium lauryl sulfate; alkylarylsulfonate salts, such as calcium dodecylbenzenesulfonate; alkylphenol-alkylene oxide addition products, such as nonylphenol-C18 ethoxylate; alcohol-alkylene oxide addition products, such as tridecyl alcohol-C16 ethoxylate; soaps, such as sodium stearate; alkylnaphthalene-sulfonate salts, such as sodium dibutylnaphthalenesulfonate; dialkyl esters of sulfosuccinate salts, such as sodium di(2-ethylhexyl) sulfosuccinate; sorbitol esters, such as sorbitol oleate; quaternary amines, such as lauryl trimethylammonium chloride; polyethylene glycol esters of fatty acids, such as polyethylene glycol stearate; block copolymers of ethylene oxide and propylene oxide; salts of mono and dialkyl phosphate esters; vegetable or seed oils such as soybean oil, rapeseed/canola oil, olive oil, castor oil, sunflower seed oil, coconut oil, corn oil, cottonseed oil, linseed oil, palm oil, peanut oil, safflower oil, sesame oil, tung oil and the like; and esters of the above vegetable oils, particularly methyl esters.

Oftentimes, some of these materials, such as vegetable or seed oils and their esters, can be used interchangeably as an agricultural adjuvant, as a liquid carrier or as a surface active agent.

Other adjuvants commonly used in agricultural compositions include compatibilizing agents, antifoam agents, sequestering agents, neutralizing agents and buffers, corrosion inhibitors, dyes, odorants, spreading agents, penetration aids, sticking agents, dispersing agents, thickening agents, freezing point depressants, antimicrobial agents, and the like. The compositions of polymeric complexes of herbicidal carboxylic acids described herein may also contain other compatible components, for example, other herbicides, plant growth regulants, fungicides, insecticides, and the like and can be formulated with liquid fertilizers or solid, particulate fertilizer carriers such as ammonium nitrate, urea and the like.

Concentrate compositions are typically diluted with an inert carrier, such as water, before application. The diluted compositions usually applied to weeds or the locus of weeds generally contain 0.0001 to 1 weight percent active ingredient and preferably contain 0.001 to 0.05 weight percent.

The compositions described herein can be applied to weeds or their locus by the use of conventional ground or aerial dusters, sprayers, and granule applicators, by addition to irrigation water, and by other conventional means known to those skilled in the art.

The following Examples are presented to illustrate various aspects of the compositions described herein and should not be construed as limitations to the claims.

### Example 1. Preparation of Compositions

### Method A - Preparation of 2,4-D-PEI Complexes in Dowanol® EB

### Samples 1-4:

Using the ingredients and amounts listed in Table 1, 20 gram (g) batches of 50 weight percent (wt%) acid equivalent (AE) of 2,4-D polyethyleneimine (PEI) complexes were prepared by adding 2,4-D acid technical grade (97%) into Dowanol® EB solvent (The Dow Chemical Company; Midland, MI). Under mixing, the appropriate amount of Lupasol® G20 Waterfree (BASF; Florham Park, NJ) was added slowly to the mixture, and mixing continued until the mixture became an amber colored, clear solution. The amounts of Dowanol® EB and Lupasol® G20 Waterfree used depended on the relative weight ratio of 2,4-D acid to Lupasol® G20 Waterfree as shown in the examples listed in Table 1.

**Table 1: Preparation of Samples 1-4**

| | **Sample 1** | **Sample 2** | **Sample 3** | **Sample 4** |
|---|---|---|---|---|
| 2,4-D acid to Lupasol® G20 Waterfree, weight ratio | 3:1 | 4:1 | 5:1 | 6.6:1 |
| 2,4-D technical acid (97% w/w) (g) | 10.3 | 10.3 | 10.3 | 10.3 |
| Lupasol® G20 Waterfree (g) | 3.3 | 2.5 | 2 | 1.5 |
| Dowanol® EB (g) | 6.4 | 7.2 | 7.7 | 8.2 |
| Total (g) | 20.0 | 20.0 | 20.0 | 20.0 |

### Samples 5-8:

The 50% acid equivalent (AE) w/w concentrates of the 2,4-D PEI complexes listed above (i.e., Samples 1-4) can be further formulated into 38% AE w/w 2,4-D PEI emulsifiable concentrate formulations by adding the appropriate amount of surfactant emulsifiers such as Atlas™ G5000 and Atlox™ 4914 (Croda Inc.; Edison, NJ). Table 2 below lists examples of such formulations as Samples 5-8. Samples 5-8 readily form homogeneous and stable emulsions upon dilution in water useful in broadcast spray applications.

**Table 2: Preparation of Samples 5-8**

| | **Sample 5** | **Sample 6** | **Sample 7** | **Sample 8** |
|---|---|---|---|---|
| 2,4-D acid to Lupasol® G20 weight ratio | 3:1 | 4:1 | 5:1 | 6.6:1 |
| 50% AE concentrate | 3.8 g of Sample 1 | 3.8 g of Sample 2 | 3.8 g of Sample 3 | 3.8 g of Sample 4 |
| Atlas G5000 | 0.5 g | 0.5 g | 0.5 g | 0.5 g |
| Atlox 4914 | 0.25 g | 0.25 g | 0.25 g | 0.25 g |
| Dowanol® EB | 0.45 g | 0.45 g | 0.45 g | 0.45 g |
| Total Weight | 5.00 g | 5.00 g | 5.00 g | 5.00 g |

### Sample 9:

Using the quantities shown in Table 3, 2,4-D acid was added to Dowanol® EB to form a white suspension in a yellow solution to which Lupasol® FG (BASF; Florham Park, NJ) was then added. The mixture was stirred and the solids dissolved to form an orange solution (Sample 9 in Table 3).

**Table 3: Preparation of Sample 9**

| **Ingredients** | **Sample 9** |
|---|---|
| 2,4-D | 50 |
| Lupasol® FG | 10 |
| Dowanol® EB | 40.00 |
| Total (g) | 100.00 |

### Method B - Preparation of a 2,4-D-PEI Complex in Water and Formulation into Two Emulsifiable Concentrates

### Sample 10:

A 200 g sample of 25% w/w Lupasol® G20 solution in water was prepared by mixing 100 g of Lupasol® G20 (50% w/w in water) with 100 g of deionized (DI) water in an appropriate container under agitation. Under rigorous agitation, 154.6 g of 2,4-D acid technical (97% w/w) was slowly added into the 25% w/w Lupasol® G20 solution. The resulting mixture was stirred for at least one day until all solid particles dissolved. The stirring was stopped and the mixture was allowed to sit for at least one day until it separated into two clear liquid layers. The 2,4-D PEI complex was isolated by separating the thick, amber colored liquid at the bottom from the top water layer. The complex prepared in this manner contained 52.7% AE w/w of 2,4-D with a 3:1 2,4-D acid to PEI on a dry weight basis.

### Sample 11:

A formulated sample of the 2,4-D Lupasol® G20 complex was prepared by mixing together 30 g of Sample 10, 9 g of Dowanol® EB, 4.6 g of Atlas™ G5000, and 2.3 g of Atlox™ 4914 to obtain a clear emulsifiable concentrate formulation of the 2,4-D Lupasol® G20 complex containing 34.5% AE w/w of 2,4-D. Sample 11 readily formed a homogeneous and stable emulsion upon dilution in water useful in broadcast spray applications.

### Sample 12:

The 2,4-D PEI complex isolated above as Sample 10 was dried under vacuum at 70 °C overnight to reach an assay of 72.8% AE of 2,4-D. 1.25 g of the dried 2,4-D PEI complex (72.8% AE) was dissolved in 2.5 g of a cyclohexanone-acetone mixture (50:50, w/w) and to this solution, 2.1 g of Pluronic® P104 (BASF; Florham Park, NJ) and 0.5 g of Soprophor® 729/P (Rhodia Inc.; Cranbury. NJ) were added, followed by a drop of Break-Thru® AF 9903 (Evonik Industries (Parsippany, NJ). The mixture was heated at 60 °C overnight to form a clear solution which was then cooled and poured into 155 g of DI water under rigorous agitation for 10 min to prepare a stable, homogenous emulsion.

### Method C - Preparation of a 2,4-D-PEI Complex in a Methanol-Water Mixture

### Sample 13:

A 214.3 g solution in methanol containing 35% AE w/w of 2,4-D acid was prepared by dissolving 77.3 g of 2,4-D acid technical (97% w/w) in 137 g of methanol in an appropriate container under agitation. Under rigorous agitation, 50 g of Lupasol® G20 (50% w/w in water) was slowly added into the methanol solution of 2,4-D. The mixture was stirred for at least one day until all solid particles dissolved and then 200 g of additional water was added and mixed well. The stirring was stopped and the mixture was allowed to sit for at least one day until it separated into two clear liquid layers. The 2,4-D PEI complex was isolated by separating the thick, amber colored liquid at the bottom from the top water/methanol layer.

### Method D - Preparation of MCPA Acid-PEI Complex using Lupasol® FG

### Samples 14 & 15:

Using the quantities shown in Table 4 for the preparation of Samples 14 and 15, Lupasol® FG ( >98 wt%) was dissolved in Dowanol® EB to form a colorless solution, to which MCPA acid solid was then added. The mixture was stirred and the solid MCPA acid gradually dissolved to form a dark yellow to dark orange solution, depending on the MCPA acid loading.

**Table 4: Preparation of Samples 14 & 15**

| **Ingredients** | **Sample 14** | **Sample 15** |
|---|---|---|
| MCPA acid | 2.50 g | 5.00 g |
| Lupasol® FG | 0.50 g | 1.00 g |
| Dowanol® EB | 7.00 g | 4.00 g |
| Total | 10.00 g | 10.00 g |

### Samples 16 & 17:

To 4.25 g each of the MCPA acid-PEI complex solutions Sample 14 and Sample 15 prepared above were added 0.25 g Ninate® 60E (Stepan Company; Northfield, IL), 0.25 g Soprophor® TS/54 (Rhodia; Cranbury, NJ), and 0.25 g Termul® 203 (Huntsman Corporation; Salt Lake City, Utah). The mixtures were heated in a microwave oven for 5 seconds to form clear yellow (Sample 16) or orange (Sample 17) solutions of the formulated samples shown in Table 5 which readily form homogeneous and stable emulsions upon dilution in water useful in broadcast spray applications.

**Table 5: Preparation of Samples 16 & 17**

| **Ingredients** | **Sample 16** | **Sample 17** |
|---|---|---|
| MCPA acid | 1.06 g | 2.13 g |
| Lupasol® FG | 0.21 g | 0.43 g |
| Dowanol® EB | 2.98 g | 1.70 g |
| Ninate® 60E | 0.25 g | 0.25 g |
| Soprophor® TS/54 | 0.25 g | 0.25 g |
| Termul® 203 | 0.25 g | 0.25 g |
| Total | 5.00 g | 5.00 g |

### Sample 18:

To 4.26 g of the MCPA acid-PEI complex solution of Sample 15 was added 0.50 g Atlas® G5000 and 0.25 g Atlox® 4914. The mixture formed was stirred to form a clear orange solution (Sample 18) of the formulated sample shown in Table 6 which readily forms a homogeneous and stable emulsion upon dilution in water useful in broadcast spray applications.

**Table 6: Preparation of Sample 18**

| **Ingredients** | **Sample 18** |
|---|---|
| MCPA acid | 2.13 g |
| Lupasol® FG | 0.43 g |
| Dowanol® EB | 1.70 g |
| Atlas® G5000 | 0.50 g |
| Atlox® 4914 | 0.25 g |
| Total | 5.00 g |

### Method E - Preparation of Triclopyr Acid-PEI Complexes using Lupasol® FG

### Samples 19-22:

Using the quantities shown in Table 7 for the preparation of Samples 19, 20, 21, and 22, Lupasol® FG was dissolved in Dowanol® EB (or methanol for Sample 22) to form colorless solutions to which the required amount of triclopyr acid was then added as a solid. The mixtures were stirred and the solid gradually dissolved to form solutions of Samples 19, 20, 21, and 22.

**Table 7: Preparation of Samples 19-22**

| **Ingredients** | **Sample 19** | **Sample 20** | **Sample 21** | **Sample 22¹** |
|---|---|---|---|---|
| Triclopyr acid | 2.50 g | 3.50 g | 5.00 g | 5.00 g |
| Lupasol® FG | 0.50 g | 0.70 g | 1.00 g | 1.00 g |
| Dowanol® EB | 7.00 g | 5.80 g | 4.00 g | 4.00 g¹ |
| Total | 10.00 g | 10.00 g | 10.00 g | 10.00 g |

| | | | | |
|---|---|---|---|---|
| ¹ Sample 22 was prepared with methanol instead of Dowanol® EB as the solvent. | | | | |

### Samples 23-25:

To 4.25 g samples each of triclopyr acid-PEI complex Samples 19 - 21 were added 0.50 g Atlas® G5000 and 0.25 g Atlox® 4914. The resulting mixtures were stirred to form homogeneous orange solutions of the formulated samples (Samples 23 - 25 in Table 8) which readily form homogeneous and stable emulsions upon dilution in water useful in broadcast spray applications.

**Table 8: Preparation of Samples 23-25**

| **Ingredients** | **Sample 23** | **Sample 24** | **Sample 25** |
|---|---|---|---|
| Triclopyr acid | 1.06 g | 1.49 g | 2.13 g |
| Lupasol® FG | 0.21 g | 0.30 g | 0.43 g |
| Dowanol® EB | 2.98 g | 2.47 g | 1.70 g |
| Atlas® G5000 | 0.50 g | 0.50 g | 0.50 g |
| Atlox® 4914 | 0.25 g | 0.25 g | 0.25 g |
| Total | 5.00 g | 5.00 g | 5.00 g |

### Method F - Preparation of Fluroxypyr Acid-PEI Complex using Lupasol® FG

### Sample 26:

Using the ingredients and amounts shown in Table 9, Lupasol® FG was dissolved in Dowanol® EB to form a colorless solution, to which fluroxypyr acid solid was then added. The mixture was stirred and the solid gradually dissolved to form a brown solution (Sample 26).

**Table 9: Preparation of Sample 26**

| **Ingredients** | **Wt%** | **Sample 26** |
|---|---|---|
| Fluroxypyr acid | 24.75 | 2.50 g |
| Lupasol® FG | 7.43 | 0.75 g |
| Dowanol® EB | 67.82 | 6.85 g |
| Total | 100.00 | 10.10 g |

### Sample 27:

To 4.25 g of fluroxypyr acid-PEI complex solution Sample 26 was added 0.25 g Ninate® 60E, 0.25 g Soprophor® TS/54, and 0.25 g Termul® 203. The mixture was heated in a microwave oven for 5 seconds to form a clear, amber colored solution of the formulated sample (Sample 27 in Table 10). Sample 27 readily forms a homogeneous and stable emulsion upon dilution in water useful in broadcast spray applications.

**Table 10: Preparation of Sample 27**

| **Ingredients** | **Wt%** | **Sample 27** |
|---|---|---|
| Fluroxypyr acid | 21.04 | 1.05 g |
| Lupasol® FG | 6.31 | 0.32 g |
| Dowanol® EB | 57.65 | 2.88 g |
| Ninate® 60E | 5.00 | 0.25 g |
| Soprophor® TS/54 | 5.00 | 0.25 g |
| Termul® 203 | 5.00 | 0.25 g |
| Total | 100.00 | 5.00 g |

### Example 2. Measurement of the Conductivity of Aqueous Solutions of the Compositions Described Herein

The conductivity of samples containing the various compositions of 2,4-D were measured using a Con 6/TDS 6 hand-held conductivity/TDS meter from Eutech Instruments Pte Ltd (available in U.S. from Oakton Instruments; Vernon Hills, IL) and are reported as millisiemens per centimeter (mS/cm). Table 11 lists the solutions used for conductivity measurements which involved first preparing concentrates in either water (Comparative Solutions A and E) or Dowanol® DB (Example Solutions 1a and 2a) and then diluting these concentrates in water to prepare the additional samples shown in Table 11. Example Solutions 1b, 1c, 2b, and 2c were measured for conductivity while fully homogeneous, but separated over time into two layers upon standing without mixing.

Example Solution 1a was prepared using Sample 3 from Example 1. Specifically, Example Solution 1a is the 50 wt% (2,4-D AE) concentrate of Sample 3 as shown in Table 11. The Example Solution 1a concentrate was used to prepare Example Solution 1b (20 wt% 2,4-D AE) and Example Solution 1c (10 wt% 2,4-D AE) shown in Table 11 by dilution in water. Example Solutions 1a, 1b, and 1c were used in the conductivity measurements as described and the results of those measurements are shown in Table 11. **Figure 1** shows images of Solutions 1a, 1b, and 1c.

Example Solution 2a was prepared using Sample 9 from Example 1. Specifically, Example Solution 2a is the 50 wt% (2,4-D AE) concentrate of sample 9 as shown in Table 11. Example Solution 2a concentrate was used to prepare Example Solution 2b (20 wt% 2,4-D AE) and Example Solution 2c (10 wt% 2,4-D AE) in Table 11 by dilution in water. Example Solutions 2a, 2b, and 2c were used in conductivity measurements as described herein and the results of these measurements are shown in Table 11. **Figure 2** shows images of Solutions 2a, 2b, and 2c.

Comparative Solutions A - D were mixtures of 2,4-D DMA and Lupasol® G20 in water at 50 wt% 2,4-D AE and 10 wt% polyamine (i.e., DMA and Lupasol® G20 combined) (Comparative Solution A); 40 wt% 2,4-D AE and 8 wt% polyamine (i.e., DMA and Lupasol® G20 combined) (Comparative Solution B); 20 wt% 2,4-D AE and 4 wt% polyamine (i.e., DMA and Lupasol® G20 combined) (Comparative Solution C); and 10 wt% 2,4-D AE and 2 wt% polyamine (i.e., DMA and Lupasol® G20 combined) (Comparative Solution D) levels. **Figure 3** shows images of Comparative Solutions B, C, and D.

Comparative Solutions E - G were mixtures of 2,4-D and TEPA (tetraethylenepentamine) in water at 21.1 wt% 2,4-D AE and 6.5 wt% polyamine (Comparative Solution E); 20 wt% 2,4-D AE and 6.16 wt% polyamine (6.16 wt% of TEPA) (Comparative Solution F); and 10 wt% 2,4-D AE and 3.08 wt% polyamine (3.08 wt% TEPA) (Comparative Solution G) levels. **Figure 4** shows images of Comparative Solutions E, F, and G.

As shown by the conductivity results in Table 11, the aqueous samples containing the compositions described herein (i.e., Example Solutions 1b, 1c, 2b, and 2c) showed significantly lower conductivity than the comparative samples (i.e., Comparative Solutions B-D and E-G). Note that that the carboxylic acid salts used in Comparative Solutions B - G were readily soluble in water (as shown in **Figures 3** and **4**), i.e., they did not form emulsions further illustrating the differences between the complexes formed using the compositions described herein and the soluble salts previously known.

**Table 11: Conductivity Measurements of 2,4-D Samples Containing Tetraethylenepentamine (TEPA) or the Polyethylenimines Lupasol® G20 or Lupasol® FG**

| | | **Example Solutions 1a-1c** | | **Example Solutions 2a-2c** | | **Comparative Solutions A-D** | | **Comparative Solutions E-G** | |
|---|---|---|---|---|---|---|---|---|---|
| *Composition* | | *2,4-D : Lupasol*® *G20 Complex* | | *2,4-D : Lupasol*® *FG Complex* | | *2,4-D DMA* + *Lupasol*® *G20 in water* | | *2,4-D DMA* + *TEPA in water* | |
| **2,4-D (AE wt%)** | **Polyamine (wt%)** | **Solution** | **Conductivity (mS/cm)** | **Solution** | **Conductivity (mS/cm)** | **Solution** | **Conductivity (mS/cm)** | **Solution** | **Conductivity (mS/cm)** |
| 50 | 10 | **1a** | 0.032 | **2a** | 0.00333 | **A** | 7.27 | | |
| 40 | 8 | | | | | **B** | 15.57 | | |
| 21.1 | 6.5 | | | | | | | **E** | 7.96 |
| 20 | 4 | **1b** | 0.704 | **2b** | 1.048 | **C** | 24.1 | **F**¹ | 7.861 |
| 10 | 2 | **1c** | 0.579 | **2c** | 0.872 | **D** | 16.59 | **G**¹ | 6.782 |

| | | | | | | | | | |
|---|---|---|---|---|---|---|---|---|---|
| ¹Comparative Solutions F and G were prepared by serial dilution of Comparative Solution E in water and contain 6.16 wt% TEPA (Comparative Solution F, 20 wt% 2,4-D AE) and 3.08 wt% TEPA (Comparative Solution G, 10 wt% 2,4-D AE), respectively. | | | | | | | | | |

In addition, Table 12 includes data showing that including 8 wt% Dowanol® DB in Comparative Solutions H and I (solutions in water according to Table 12) had only a small affect on their conductivity as they had significantly larger conductivity values compared to Example Solution 1c.

**Table 12: Conductivity Measurements of 10 Wt% AE 2,4-D Aqueous Samples Containing 8Wt% Dowanol® EB, and Lupasol® G20, or TEPA**

| Composition | **2,4-D Solutions¹** | | |
|---|---|---|---|
| | **Comparative Solution H** | **Comparative Solution I** | **Example Solution 1c** |
| | 2,4-D DMA + Lupasol® G20 | 2,4-D TEPA | 2,4-D : Lupasol® G20 Complex |
| 2,4-D wt% AE | 10% | 10% | 10% |
| wt% Dowanol EB | 8% | 8% | 8% |
| Conductivity (mS/cm) | 13.68 | 6.00 | 0.579 |

| | | | |
|---|---|---|---|
| ¹ Comparative Solution H and Example Solution 1c each contain 2 wt% of Lupasol® G20 polyethylenimine and Comparative Solution I contains 3.08 wt% of TEPA (tetraethylenepentamine). | | | |

### Example 3. Determination of Relative Volatility by Thermo-gravimetric Analysis

Using a TA Instruments (New Castle, DE) Model TGA 2050 thermo-gravimetric analyzer, a typical thermo-gravimetric analysis (TGA) is carried out by (a) weighing out 10-20 mg of the sample, (b) loading it into an aluminum or platinum holding pan, and (c) measuring and recording the mass loss of the sample over time at a constant temperature of 120 °C. The volatility of the sample is obtained as the rate of mass loss per unit time when the loss rate becomes constant (reaches steady state).

Table 13 shows the relative volatility of various 2,4-D and triclopyr samples. Relative volatility is defined as the ratio of the volatility of a given 2,4-D or triclopyr sample over that of the corresponding 2,4-D DMA or triclopyr triethylamine salt that was arbitrarily set at 100. As shown in the Table 13, the 2,4-D PEI complex made from Lupasol® G20 (Run 4) demonstrated significantly lower volatility than the corresponding 2,4-D acid, the 2,4-D ethylhexyl ester, or the 2,4-D dimethylamine (DMA) salt. In addition, the triclopyr PEI complex made from Lupasol® FG (Run 8, Table 13) demonstrated significantly lower volatility than the corresponding triclopyr acid, the triclopyr butoxyethyl ester, or the triclopyr triethylamine salt.

**Table 13: Volatility Data**

| **Run #** | **2,4-D Sample** | **Relative volatility** |
|---|---|---|
| 1 | 2,4-D ethylhexyl ester | 1201 |
| 2 | 2,4-D acid tech | 249 |
| 3 | 2,4-D DMA salt | 100 |
| 4 | 2,4-D Lupasol® G20 complex (Sample 10) | 22 |
| 5 | triclopyr acid | 135 |
| 6 | triclopyr butoxyethyl ester | 302 |
| 7 | triclopyr triethylamine salt | 100 |
| 8 | triclopyr-Lupasol FG complex (Sample 22) | 16 |

### Example 4. Soil Binding

The three samples described in Table 14 were individually diluted in water to form ca. 500 ppm solutions. To 50 g of each diluted sample was added 25 g of Midwest soil. The soil/water mixtures were shaken at 220 rpm for 16 h at room temperature on an IKA®-Werke KS-501 digital platform shaker (IKA® Works, Inc.; Wilmington, NC). After centrifuging the samples at 2500 rpm for 15 min, the supernatant liquid in each sample was filtered through 0.45 micron (µm) membrane filter and acidified to pH 1 before analyzing for active ingredient (AI) content by high performance liquid chromatography (HPLC) analysis. The weight of 2,4-D that remained bound to the soil for each sample is shown in Table 14. As can be seen from the results in Table 14, the compositions of Sample 7 and Sample 9 were more tightly bound to the soil than the DMA® 6 Weed Killer.

**Table 14: Soil Binding Results**

| **Sample** | **Description** | **µg AI/g soil** |
|---|---|---|
| Sample 9 | 50 Wt% AE 2,4-D Lupasol FG complex | 109 |
| Sample 7 | 38 Wt% AE 2,4-D Lupasol G20 complex | 135 |
| DMA® 6 Weed Killer¹ | 61.4 Wt% AE 2,4-D DMA | 24 |

| | | |
|---|---|---|
| ¹ Dow AgroSciences LLC (Indianapolis, IN). | | |

### Example 5. Weed Control in Greenhouse Tests

Plant Propagation: A peat based potting soil, Metro-mix® 360 (Sun Gro Horticulture Canada CM Ltd.; Vancouver, British Columbia), was used as the soil media for this test. Several seeds of each species were planted in 10 cm square pots and top watered twice daily. Weed species were propagated in the greenhouse at a constant temperature of 26 to 28 °C and 50 to 60% relative humidity. Natural light was supplemented with 1000-watt metal halide overhead lamps with an average illumination of 500 microEinsteins (µE) m⁻² s⁻¹ photosynthetic active radiation (PAR). The photoperiod was 16 hr. Plant material was top-watered prior to treatment and sub-irrigated after treatment.

Application of Treatments: Treatments were applied with a track sprayer manufactured by Allen Machine Works (Jonesboro, TN). The sprayer utilized an 8002E spray nozzle, spray pressure of 262 kPa, and speed of 1.8 mph to deliver 187 L/Ha. The nozzle height was 46 cm above the plant canopy. The growth stage of the various weed species ranged from 2 to 4 leaf. Treatments were replicated 3 times. Plants were returned to the greenhouse after treatment and sub-watered throughout the duration of the experiment. Plant material was fertilized twice weekly with Hoagland's fertilizer solution. Visual assessments of percent control were made on a scale of 0 to 100% as compared to the untreated control plants (where 0 is equal to no control and 100 is equal to complete control). The weed plant species used in these tests are CASOB (Sicklepod) and CHEAL (Common lambsquarters) unless noted otherwise.

Results: Tables 15, 16, 17, and 18 show comparative herbicidal activity data for representative applications of the compositions described herein and comparative commercial products. The commercial products used in these tests were: (1) DMA® 4 IVM, a 38.64 wt% AE (456 g/L) concentrate of 2,4-D dimethylammonium (DMA) in water; (2) Esteron®, a 600 g/L AE 2,4-D emulsifiable concentrate of 2,4-D ethylhexyl ester; (3) Garlon® 4, a 44.3 wt% (480 g/L) AE emulsifiable concentrate of triclopyr butoxyethyl ester; and (4) MCPA Ester 600 Liquid Herbicide, a 54.15 wt% (600 g/L) AE emulsifiable concentrate of MCPA ethylhexyl ester (Nufarm USA; Burr Ridge, IL). The DMA®, Esteron®, and Garlon® products are available from Dow AgroSciences LLC (Indianapolis, IN)).

**Table 15: Herbicidal Activity**

| **Herbicide Active Ingredient** | **Description of Treatment** | **Rate (g AE/ha)** | **% Control CASOB** | **% Control CHEAL** |
|---|---|---|---|---|
| 2,4-D DMA | DMA® 4 IVM | 100 | 30 | 38 |
| 2,4-D PEI | Sample 7 | 100 | 40 | 70 |
| 2,4-D EHE | Esteron® | 100 | 40 | 80 |
| 2,4-D DMA | DMA® 4 IVM | 200 | 37 | 80 |
| 2,4-D PEI | Sample 7 | 200 | 53 | 87 |
| 2,4-D EHE | Esteron® | 200 | 52 | 95 |
| Triclopyr BEE | Garlon® 4 | 280 | 73 | 82 |
| Triclopyr PEI | Sample 23 | 280 | 75 | 72 |
| Triclopyr BEE | Garlon® 4 | 560 | 84 | 85 |
| Triclopyr PEI | Sample 23 | 560 | 93 | 82 |

**Table 16: Herbicidal Activity**

| Herbicide Active Ingredient | Description of Treatment | Rate (g AE/ha) | % Control CASOB | % Control CHEAL |
|---|---|---|---|---|
| MCPA EHE | MCPA Ester 600 | 140 | 33 | 72 |
| MCPA PEI | Sample 16 | 140 | 48 | 83 |
| MCPA EHE | MCPA Ester 600 | 280 | 40 | 85 |
| MCPA PEI | Sample 16 | 280 | 74 | 95 |
| 2,4-D PEI | Sample 12 | 100 | 68 | 95 |
| 2,4-D EHE | Esteron® | 100 | 50 | 88 |
| 2,4-D PEI | Sample 12 | 200 | 85 | 99 |
| 2,4-D EHE | Esteron® | 200 | 77 | 95 |

**Table 17: Herbicidal Activity**

| **Herbicide Active Ingredient** | **Description of Treatment** | **Rate (g AE/ha)** | **% Control CASOB** | **% Control CHEAL** |
|---|---|---|---|---|
| 2,4-D DMA | DMA® 4 IVM | 100 | 40 | 50 |
| 2,4-D EHE | Esteron® | 100 | 43 | 95 |
| 2,4-D PEI | Sample 5 | 100 | 65 | 96 |
| 2,4-D PEI | Sample 6 | 100 | 52 | 95 |
| 2,4-D PEI | Sample 7 | 100 | 47 | 96 |
| 2,4-D PEI | Sample 8 | 100 | 47 | 95 |
| 2,4-D DMA | DMA® 4 IVM | 200 | 70 | 94 |
| 2,4-D EHE | Esteron® | 200 | 73 | 97 |
| 2,4-D PEI | Sample 5 | 200 | 77 | 99 |
| 2,4-D PEI | Sample 6 | 200 | 73 | 97 |
| 2,4-D PEI | Sample 7 | 200 | 87 | 99 |
| 2,4-D PEI | Sample 8 | 200 | 47 | 100 |

**Table 18: Herbicidal Activity**

| **Herbicide Active Ingredient** | **Description of Treatment** | **Rate (g AE/ha)** | **% Weed Control 14 days After Treatment¹** |
|---|---|---|---|
| 2,4-D EHE | Esteron® | 200 | 79% |
| 2,4-D DMA | DMA® 4 IVM | 200 | 66% |
| 2,4-D PEI | Sample 11 | 200 | 81% |

| | | | |
|---|---|---|---|
| ¹ % Weed control value is obtained by the Least Square Mean of the % control of five weed species (Sicklepod, Common Lambsquarters, Velvetleaf, Prickly Sida, and Common Ragweed) with 3 replicates per treatment. | | | |

### Example 6. Reduction of Eye Irritation

Eye irritation testing was done according to guideline testing requirements specified in: (1) OECD Guideline for the Testing of Chemicals, Procedure 405 (2002), (2) U.S. EPA Health Effects Test Guidelines, OPPTS 870.2400 (1998), (3) JMAFF 12 -Nouan-1847 (2000) and (4) Official Journal of the European Communities, Methods for the Determination of Toxicity, Part B.5 (Eye Irritation), Directive 2004/73/ED, 29 April 2004.

Test material formulations were administered to New Zealand albino rabbit (1 to 3 animals per formulation) to determine the potential to produce eye irritation. The material was applied in a single dose to the conjuctival sac of one eye of each animal. The other eye remained untreated and served as a control. Irritation of the cornea, iris and conjunctivae were evaluated 21 days after application of the test material. Maximum irritation scores to be obtained are 4 for corneal opacity, 2 for iritis and 10 for conjunctivitis (these values do not take into account the area of cornea evaluated or additional factors used in the calculations).

The results are reported as irritation present in the cornea, iris and conjunctivae in the most sensitive animal 21 days after application. Testing was conducted with the following concentrates: (1) 2,4-D PEI complex (Lupasol® G20 complex, 456 grams acid equivalent per liter (gae/1)), (2) 2,4-D DMA (dimethyl ammonium salt, 456 gae/1), (3) 2,4-D DMEA (dimethylethyl ammonium salt, 456 gae/1), (4) 2,4-D IPA (isopropyl ammonium salt, 456 gae/1), and (5) 2,4-D TIPA (triisopropanol ammonium salt, 456 gae/1). As shown in Table 19, the 2,4-D PEI complex caused little or no eye irritation when evaluated 14 days after application compared to the other 2,4-D formulations that exhibited significant eye irritation when evaluated 21 days after application.

**Table 19: Eye Irritation Scores for 2,4-D Formulations 21 Days After Application of the Test Material**

| **2,4-D Formulation** | **2,4-D gae/L¹** | **Cornea** | **Iris** | **Conjunctivae** |
|---|---|---|---|---|
| 2,4-D PEI (Sample 7 in Table 2)² | 456 | 0 | 0 | 0³ |
| 2,4-D DMA | 456 | 3 | 1 | 3 |
| 2,4-D DMEA | 456 | 4 | 2 | 6 |
| 2,4-D IPA | 456 | 4 | 0 | 6 |
| 2,4-D TIPA | 456 | 3 | 0 | 1 |

| | | | | |
|---|---|---|---|---|
| ¹ grams acid equivalent per liter; the 2,4-D salt concentrates were formulated as aqueous solutions while the 2,4-D PEI complex was dissolved in Dowanol EB (Butyl Cellosolve); ² eye irritation was evaluated 14 days after application; ³ conjunctivae evaluation utilized a 0, 1, 2, 3 point system with 0 meaning the blood vessels in the tested eye were normal. | | | | |

### Example 7. Spray Drift Reduction

### Preparation of 2,4-D-PEI Complexes in 2-Propoxyethanol:

### Sample 28:

Using the ingredients and quantities shown in Table 20, Lupasol® FG ( >98 wt%) was dissolved in 2-propoxyethanol (Acros Organics) to form a colorless solution, to which 2,4-D acid solid was then added. The mixture was stirred and the solid acid gradually dissolved to form a brown solution (Sample 28).

**Table 20: Preparation of Sample 28**

| **Ingredients** | **Sample 28** |
|---|---|
| 2,4-D acid | 25.00 g |
| Lupasol® FG | 5.00 g |
| 2-Propoxyethanol | balance |
| Total | 50.00 g |

### Sample 29:

To 8.03 g of the 2,4-D-PEI complex solution Sample 28 prepared above were added 0.95 g Termul® 203 (Huntsman Corporation; Salt Lake City, Utah), 0.40 g Makon TD-3 (Stepan Company; Northfield, IL), and 0.62 g 2-propoxyethanol. The mixture was heated in a microwave oven for 5 seconds to form a clear brown solution of the formulated sample shown in Table 21 which readily formed a homogeneous and stable emulsion upon dilution in water for use in broadcast spray applications.

### Sample 30:

To 5 g Sample 29 was added 5 g 2-proxyethanol. A yellow solution (Sample 30 in Table 21) was formed upon shaking, which readily formed a homogeneous and stable emulsion upon dilution in water for use in broadcast spray applications.

**Table 21: Preparation of Samples 29 & 30**

| **Ingredients** | **Sample 29** | **Sample 30** |
|---|---|---|
| 2,4-D acid | 4.01 g | 2.01 g |
| Lupasol® FG | 0.80 g | 0.40 g |
| Termul® 203 | 0.95 g | 0.48 g |
| Makon TD-3 | 0.40 g | 0.20 g |
| 2-Proxyethanol | balance | balance |
| Total | 10.00 g | 10.00 g |

### Preparation of spray solutions and spray droplet analysis:

Two spray solutions containing 2,4-D-PEI complexes for spray application at a use rate of 400 grams acid equivalent per hectare (gae/ha) and at spray volumes of 20 gallons per acre (GPA) were prepared by diluting 2.13 g of Sample 29 and 4.26 g of Sample 30 each into deionized water to provide a total volume of 400 mL of each spray solution. A control spray solution was prepared with DMA® 4 IVM (aqueous concentrate containing 38.4 wt% AE of 2,4-D DMA salt; Dow AgroSciences; Indianapolis, IN) for spray application at a use rate of 400 gae/ha and at a spray volume of 20 GPA. All three spray solutions were lightly shaken by hand until each sample was homogenous. The spray solutions were sprayed using a Teejet® 8002 flat fan nozzle (Teejet Technologies; Wheaton, IL) at 40 psi (276 kiloPascal) and the spray droplet size distribution measurement was made with a Sympatec Helos/KF high resolution laser diffraction particle sizer with an R7 lens (Sympatec GmbH; Clausthal-Zellerfeld, Germany). The tip of the nozzle was situated 12 inches (30.5 centimeters) above the path of the laser beam of the Sympatec particle sizer. The percentage of driftable fines was expressed as the volume percentage of spray droplets below 150 µm volume mean diameter (VMD) as shown in Table 22.

**Table 22: Spray Droplet Analysis**

| **Sample** | **Herbicide Spray Droplet Analysis** | |
|---|---|---|
| | **Spray Droplet VMD, µm** | **Volume Percentage of Driftable Fines <150 µm VMD** |
| DI water | 170 | 43% |
| DMA® 4 IVM | 166 | 44% |
| Sample 29 | 228 | 24% |
| Sample 30 | 201 | 33% |

### Example 8. Increased Binding to Plant Surfaces

### Preparation of 2,4-D-PEI Complexes in Cyclohexanone:

### Sample 31:

Sample 31 (Table 23) was prepared as described for Sample 28 except that cyclohexanone was used in place of 2-propoxyethanol as the solvent.

**Table 23: Preparation of Sample 31**

| **Ingredients** | **Sample 31** |
|---|---|
| 2,4-D acid | 25.00 g |
| Lupasol® FG | 5.00 g |
| cyclohexanone | balance |
| Total | 50.00 g |

### Sample 32:

To 32.11 g the 2,4-D-PEI complex solution Sample 31 prepared above were added 3.80 g Atlas G5000 (Croda), 1.60 g Makon TD-3 (Stepan Company; Northfield, IL), and 2.49 g cyclohexanone. The mixture was heated in a microwave oven for 5 seconds to form a clear brown solution of the formulated sample shown in Table 24 which readily formed a homogeneous and stable emulsion upon dilution in water for use in broadcast spray applications.

### Sample 33:

To a 15 g of Sample 32 was added 12.98 g cyclohexanone, 1.43 g Atlas G5000, and 0.60 g Makon TD-3. A yellow solution of the formulated sample shown in Table 24 was formed upon heating the mixture in a microwave oven for 5 seconds which readily formed a homogeneous and stable emulsion upon dilution in water for use in broadcast spray applications.

**Table 24: Preparation of Samples 32 & 33**

| **Ingredients** | **Sample 32** | **Sample 33** |
|---|---|---|
| 2,4-D acid | 16.06 g | 6.02 g |
| Lupasol® FG | 3.21 g | 1.20 g |
| Atlas G5000 | 3.80 g | 2.86 g |
| Makon TD-3 | 1.60 g | 1.20 g |
| cyclohexanone | balance | balance |
| Total | 40.00 g | 30.00 g |

### Spray study:

Formulated Samples 32 and 33 (Table 24) and Sample 7 were diluted in water and sprayed on plants at a use rate of 400 g ae/ha of the 2,4-D PEI complex and a spray volume of 20 GPA. After spraying, the plants were allowed to dry and leaf disks with a diameter of one inch were punched out of the sicklepod and cabbage plants (10 disks for each plant species) and the velvetleaf and soybean plants (15 disks for each plant species). The leaf disks were each washed with 15 mL acetonitrile for 10 seconds. Three replicates were conducted for each plant species. The washes were filtered and analyzed by HPLC for 2,4-D (ae basis). As shown in Table 25, the three PEI complexes displayed improved binding to the plant surfaces in comparison to the 2,4-D DMA salt formulation on sicklepod, soybean, and cabbage plants and showed similar or decreased binding to velvetleaf plants.

**Table 25: Plant Binding Study - Sprayed Samples**

| **Formulation** | **Active** | **2,4-D from Leaf Washes (µg AE/in² leaf)** | | | |
|---|---|---|---|---|---|
| | | **Velvetleaf** | **Sicklepod** | **Soybean** | **Cabbage** |
| DMA® 6 Weed Killer | 2,4-D DMA | 9.68 | Not detected | 9.02 | 10.70 |
| Sample 7 | 2,4-D Lupasol G20 complex | 6.80 | 1.99 | 10.28 | 12.98 |
| Sample 32 | 2,4-D Lupasol FG complex | 9.54 | 3.14 | Not tested | Not tested |
| Sample 33 | 2,4-D Lupasol FG complex | 8.23 | 7.04 | Not tested | Not tested |

### Leaf dip study:

A plant leaf dipping method was used to study the plant surface binding and resistance to water wash off of 2,4-D PEI complex Sample 7. Stock solutions containing 1000 ppm of 2,4-D (ae basis) were prepared from 2,4-D DMA salt and 2,4-D PEI complex Sample 7 (Table 26). Wheat leaves (on fully intact plants) were submerged in each solution for 10 seconds with circular motion 5 s to the left, then 5 s to the right. The wheat plants were then hung upside down and air dried for 1.5 h. The leaves on the dried wheat plants were then removed by cutting them off approximately 1 centimeter from the stem. Some of the dried wheat plants prepared above in the leaf dip test were used in a water rinse test by submerging them in DI water for 10 min before hanging them upside down and air drying them for 1.5 h. The leaves from these dried wheat plants were harvested as described in the previous method. The harvested, dry leaves from the two tests described above were then extracted with acetonitrile as follows. 1 g of harvested wheat leaves were placed into a 20 mL vial with 10 stainless steel beads (1/8 inch diameter), followed by the addition of 5 g of acetonitrile. The vials were shaken at 220 rpm for 16 h at room temperature on an IKA-Werke KS-501 digital platform shaker (IKA Works, Inc.; Wilmington, NC). The solutions were then filtered through 0.45 micron (µm) PTFE filter membrane (Whatman) and subjected to HPLC analysis. For samples of the PEI complex, the extracted solutions were acidified to pH 1 before injection into the HPLC column. Three or four replicate analyses were conducted for each sample.

The 2,4-D PEI complex (Sample 7 in Table 26) displayed greater than 25x improvement in plant surface binding over the 2,4-D DMA salt after the leaf dip test. After the water rinse test, the 2,4-D PEI complex showed greater than 18x residual 2,4-D on the leaf surface compared to the 2,4-D DMA salt.

**Table 26: Wheat Plant Binding Study**

| **Formulation** | **Active** | **Leaf Dip Test** | **Water Rinse Test** |
|---|---|---|---|
| | | **Residual 2,4-D (µg ae/g leaf)** | **Residual 2,4-D (µg ae/g leaf)** |
| DMA® 6 Weed Killer | 2,4-D DMA | 27.00 | 13.73 |
| Sample 7 | 2,4-D PEI complex | 741.34 | 256.58 |

## Claims

1. An emulsifiable concentrate comprising, with respect to the total composition, from 10 to 50 wt.-% on an acid equivalent basis of a complex of a herbicidal carboxylic acid and an amine-containing polymer or oligomer, which is a polyethyleneimine with a molecular weight of greater than 250 and less than 2,000,000, and a nitrogen content of from 10 to 50 wt.-%, an organic solvent and optionally one or more additional inert ingredients.

2. The emulsifiable concentrate of claim 1, wherein the herbicidal carboxylic acid is an aryloxyalkanoic acid compound of the general formula 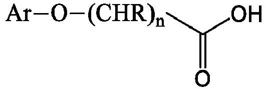 wherein R is H or CH₃, n is an integer 1, 2 or 3 and Ar is a substituted phenyl or pyridine group with one or more substituents selected from halogen, C₁-C₆ alkyl, C₁-C₆ haloalkyl, C₁-C₆ alkoxy, amino, C₁-C₆ alkylamino, and di(C₁-C₆ alkyl)amino.

3. The emulsifiable concentrate of claim 1, wherein the herbicidal carboxylic acid is 2,4-D, 2,4-DB, MCPA, MCPB, mecoprop, mecoprop-P, triclopyr, or fluroxypyr.

4. The emulsifiable concentrate of claim 1, wherein the molar ratio of amine groups to carboxylic acid groups is from 5:1 to 1:5.

5. The emulsifiable concentraten of claim 1, wherein the molar ratio of amine groups to carboxylic acid groups is from 2:1 to 1:2.

6. A diluted composition obtained by diluting the emulsifiable concentrate of claim 1 with an inert carrier.

7. A method of controlling undesirable vegetation comprising contacting the vegetation or the locus thereof with, or applying to the soil to prevent the emergence of vegetation, a herbicidally effective amount of the diluted composition of claim 6.

8. A method of preparing the emulsifiable concentrate of claim 1 comprising combining the herbicidal carboxylic acid with the amine-containing polymer or oligomer with the aid of the polar organic solvent and optionally additional inert formulation ingredients.

9. A method of reducing the volatility of a herbicidal carboxylic acid comprising preparing the emulsifiable concentrate of claim 1 according to the method of claim 8.

10. A method of reducing the soil mobility of a herbicidal carboxylic acid comprising preparing the emulsifiable concentrate of claim 1 according to the method of claim 8

11. A method for reducing the eye irritating properties of aqueous herbicidal concentrates of alkylamine salts of herbicidal carboxylic acids derived from mono-, di- or trialkylamines comprising preparing the emulsifiable concentrate of claim 1 according to the method of claim 8

12. A method for reducing spray drift of a solution containing a herbicidal carboxylic acid comprising spraying the diluted composition of claim 6.

13. A method for improving the binding to plant surfaces of alkylamine salts of herbicidal carboxylic acids derived from mono-, di-, or trialkylamines comprising applying the diluted composition of claim 6 to the plant surfaces.

## Patentansprüche

1. Ein emulgierbares Konzentrat umfassend, bezogen auf die Gesamtzusammensetzung, von 10 bis 50 Gew.-% auf einer Säureäquivalentbasis eines Komplexes einer herbiziden Carbonsäure und eines aminhaltigen Polymers oder Oligomers, welches ein Polyethylenimin mit einem Molekulargewicht von mehr als 250 und weniger als 2.000.00 und einem Stickstoffgehalt von 10 bis 50 Gew.-% ist, ein organisches Lösungsmittel und wahlweise ein oder mehrere zusätzliche inerte Inhaltsstoffe.

2. Das emulgierbare Konzentrat gemäß Anspruch 1, wobei die herbizide Carbonsäure eine Aryloxyalkansäure-Verbindung der allgemeinen Formel 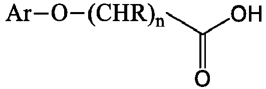 ist, wobei R H oder CH₃ ist, n eine ganze Zahl 1, 2 oder 3 ist und Ar eine substituierte Phenyl- oder Pyridingruppe mit einem oder mehreren Substituenten, ausgewählt aus Halogen, C₁-C₆-Alkyl, C₁-C₆-Halogenalkyl, C₁-C₆-Alkoxy, Amino, C₁-C₆-Alkylamino und Di(C₁-C₆ alkyl)amino, ist.

3. Das emulgierbare Konzentrat gemäß Anspruch 1, wobei die herbizide Carbonsäure 2,4-D, 2,4-DB, MCPA, MCPB, Mecoprop, Mecoprop-P, Triclopyr oder Fluroxypyr ist.

4. Das emulgierbare Konzentrat gemäß Anspruch 1, wobei das molare Verhältnis von Amingruppen zu Carbonsäuregruppen von 5:1 bis 1:5 beträgt.

5. Das emulgierbare Konzentrat gemäß Anspruch 1, wobei das molare Verhältnis von Amingruppen zu Carbonsäuregruppen von 2:1 bis 1:2 beträgt.

6. Eine verdünnte Zusammensetzung erhalten durch Verdünnen des emulgierbaren Konzentrats gemäß Anspruch 1 mit einem inerten Trägerstoff.

7. Ein Verfahren zur Bekämpfung unerwünschter Vegetation umfassend das In-Kontakt-Bringen der Vegetation oder des Ortes derselben mit einer herbizid wirksamen Menge der verdünnten Zusammensetzung gemäß Anspruch 6 oder das Anwenden derselben auf den Boden, um das Auflaufen der Vegetation zu verhindern.

8. Ein Verfahren zur Herstellung des emulgierbaren Konzentrats gemäß Anspruch 1 umfassend das Vereinen der herbiziden Carbonsäure mit dem aminhaltigen Polymer oder Oligomer mit Hilfe des polaren organischen Lösungsmittels und wahlweise zusätzlichen inerten Formulierungsinhaltsstoffen.

9. Ein Verfahren zur Verminderung der Flüchtigkeit einer herbiziden Carbonsäure umfassend das Herstellen des emulgierbaren Konzentrats gemäß Anspruch 1 gemäß dem Verfahren des Anspruchs 8.

10. Ein Verfahren zur Verminderung der Bodenmobilität einer herbiziden Carbonsäure umfassend das Herstellen des emulgierbaren Konzentrats gemäß Anspruch 1 gemäß dem Verfahren des Anspruchs 8.

11. Ein Verfahren zur Verminderung der augenreizenden Eigenschaften von wässrigen Herbizidkonzentraten von Alkylaminsalzen von herbiziden Carbonsäuren, die von Mono-, Di- oder Trialkylaminen abgeleitet sind, umfassend das Herstellen des emulgierbaren Konzentrats gemäß Anspruch 1 gemäß dem Verfahren des Anspruchs 8.

12. Ein Verfahren zur Reduktion der Sprühabdrift einer Lösung enthaltend eine herbizide Carbonsäure umfassend das Versprühen der verdünnten Zusammensetzung gemäß Anspruch 6.

13. Ein Verfahren zur Verbesserung der Anbindung von Alkylaminsalzen von herbiziden Carbonsäuren, die sich von Mono-, Di- oder Trialkylaminen ableiten, an Pflanzenoberflächen, umfassend das Anwenden der verdünnten Zusammensetzung gemäß Anspruch 6 auf die Pflanzenoberflächen.

## Revendications

1. Concentré émulsifiable comprenant, par rapport à la composition totale, de 10 à 50 % en poids sur une base d'équivalent d'acide d'un complexe d'un acide carboxylique herbicide et d'un polymère ou oligomère contenant des groupes amino, qui est une polyéthylène-imine ayant une masse moléculaire de plus de 250 et moins de 2 000 000, et une teneur en azote de 10 à 50 % en poids, un solvant organique et en option un ou plusieurs composant(s) inerte(s) supplémentaire(s).

2. Concentré émulsifiable selon la revendication 1, dans lequel l'acide carboxylique herbicide est un composé acide aryloxyalcanoïque de formule générale 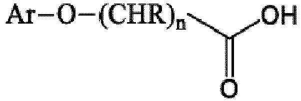 dans laquelle R est un atome d'hydrogène ou un groupe CH₃, n est un nombre entier valant 1, 2 ou 3 et Ar est un groupe phényle ou pyridine substitué, portant un ou plusieurs substituant(s) choisi(s) parmi halogéno, alkyle en C₁-C₆, halogéno-alkyle(C₁-C₆), alcoxy en C₁-C₆, amino, alkyl(C₁-C₆)amino, et di(alkyl(C₁-C₆))amino.

3. Concentré émulsifiable selon la revendication 1, dans lequel l'acide carboxylique herbicide est le 2,4-D, le 2,4-DB, le MCPA, le MCPB, le mécoprop, le mécoprop-P, le triclopyr, ou le fluroxypyr.

4. Concentré émulsifiable selon la revendication 1, dans lequel le rapport molaire des groupes amino aux groupes acide carboxylique vaut de 5:1 à 1:5.

5. Concentré émulsifiable selon la revendication 1, dans lequel le rapport molaire des groupes amino aux groupes acide carboxylique vaut de 2:1 à 1:2.

6. Composition diluée obtenue par dilution avec une substance de support inerte du concentré émulsifiable selon la revendication 1.

7. Procédé de lutte contre une végétation indésirable, comprenant le fait de mettre la végétation, ou le lieu où elle se trouve, en contact avec ou d'appliquer sur le sol pour empêcher la levée de la végétation, une quantité à effet herbicide de la composition diluée de la revendication 6.

8. Procédé de préparation du concentré émulsifiable selon la revendication 1, comprenant le fait d'associer l'acide carboxylique herbicide au polymère ou à l'oligomère contenant des groupes amino, à l'aide du solvant organique polaire, et en option des composants inertes supplémentaires de la formulation.

9. Procédé de réduction de la volatilité d'un acide carboxylique herbicide, comprenant le fait de préparer conformément au procédé de la revendication 8 le concentré émulsifiable de la revendication 1.

10. Procédé de réduction de la mobilité dans le sol d'un acide carboxylique herbicide, comprenant le fait de préparer conformément au procédé de la revendication 8 le concentré émulsifiable de la revendication 1.

11. Procédé pour réduire les propriétés d'irritation oculaire de concentrés herbicides aqueux de sels d'alkylamines d'acides carboxyliques herbicides dérivés de mono-, di- ou trialkylamines, comprenant le fait de préparer conformément au procédé de la revendication 8 le concentré émulsifiable de la revendication 1.

12. Procédé pour réduire la dérive de pulvérisation d'une solution contenant un acide carboxylique herbicide, comprenant le fait de pulvériser la composition diluée de la revendication 6.

13. Procédé pour améliorer la fixation à des surfaces végétales de sels d'alkylamines d'acides carboxyliques herbicides dérivés de mono-, di- ou trialkylamines, comprenant le fait d'appliquer sur les surfaces végétales la composition diluée de la revendication 6.
